# FASCICULE DE BREVET EUROPEEN

(11) **EP 1 839 076 B1**
(45) Date de publication et mention de la délivrance du brevet: **01.04.2009**
(21) Numéro de dépôt: 05821765.4
(22) Date de dépôt: 06.12.2005
(51) Int. Cl.: G02B 1/04, A61L 27/16

(54) **COMPOSITION A AGENT CHROMOPHORE JAUNE POUR LENTILLES INTRAOCULAIRES ET LENTILLES AINSI OBTENUES**
GELBE CHROMOPHORMITTELZUSAMMENSETZUNG FÜR INTRAOKULARE LINSEN UND SO ERHALTBARE LINSEN
YELLOW CHROMOPHORE AGENT COMPOSITION FOR INTRAOCULAR LENSES AND THE THUS OBTAINABLE LENSES

(30) Priorité: 13.01.2005 BE 200500021
(43) Date de publication de la demande: 03.10.2007
(73) Titulaire: Physiol, 4031 Angleur (BE)
(72) Inventeur: PAGNOULLE, Christophe, Robert, Marie A., B-4800 Verviers (BE); NOLET DE BRAUWERE VAN STEELAND, Marc, Emmanuel, B-1640 Rhode-saint-genèse (BE)
(74) Mandataire: Claeys, Pierre
(86) Numéro de dépôt international: PCT/EP2005/056526
(87) Numéro de publication internationale: WO 2006/074843

(56) Documents cités:
- US-B1- 6 242 551
- US-B1- 6 326 448
- PATENT ABSTRACTS OF JAPAN vol. 1998, no. 12, 31 octobre 1998 (1998-10-31) & JP 10 195324 A (HOYA CORP), 28 juillet 1998 (1998-07-28)
- JIAN PING CHEN, JIAN PING GAO, ZHI YUAN WANG: "Preparation and photochemical study of soluble optically active block copolymethacrylates and azo-containing random copolymethacrylates" JOURNAL OF POLYMER SCIENCE PART A: POLYMER CHEMISTRY, vol. 35, no. 1, 1997, pages 9-16, XP002344132 cité dans la demande

## Description

La cataracte correspond à l'opacification du cristallin de l'oeil. Elle provoque la cécité en bloquant le passage de la lumière. La cataracte est la principale cause de cécité de la population mondiale. Le seul remède actuel à la cataracte est le remplacement du cristallin opacifié par une lentille intraoculaire (LIO) réalisée dans un matériau polymère.

Le développement des lentilles intraoculaires est en perpétuelle évolution et prend en compte à la fois les exigences du chirurgien et le bénéfice fonctionnel attendu par le patient. Les principaux progrès réalisés récemment concernent la taille d'incision (nouveaux matériaux et «designs» de la LIO), la limitation de la cataracte secondaire (bords carrés et angulation des haptiques de la LIO), l'amélioration de la fonctionnalité de la LIO (lentille multifocale et accommodatrice).

La présente invention concerne une composition polymère pour lentille intraoculaire, comprenant au moins un agent chromophore jaune copolymérisé, constitué d'un premier isomère et d'un deuxième isomère et filtrant au moins une part de lumière bleue.

De nombreuses publications font état de la nocivité de la "lumière bleue" sur la rétine qui constitue un facteur important dans la genèse des lésions rétiniennes telles que la dégénérescence maculaire liée à l'âge (DMLA). Cette maladie est l'une des causes les plus fréquentes de perte visuelle sévère.

La lumière bleue (photo-toxique) joue en effet un rôle dans la dégradation photochimique des cellules épithéliales du pigment rétinien via des réactions photochimiques qui peuvent altérer les cellules photo-réceptrices, et donc la vision .

Le cristallin humain offre une protection naturelle contre ces photons délétères. Dès la 4ème année de vie, l'effet de la lumière conduit à la formation d'un pigment jaune dans le cristallin qui agit comme un filtre protecteur en réduisant la part de lumière bleue qui pénètre dans l'oeil et donc les risques de lésions rétiniennes. Ce "jaunissement" du cristallin et son effet protecteur s'accentuent avec l'âge. Ainsi à 54 ans, le cristallin ne transmet plus la part de la lumière de longueur d'onde inférieure à 400 nm, alors que la transmission des longueurs d'onde entre 400 et 500 nm est fortement diminuée.

Il va de soi que cette protection disparaît aussitôt que le cristallin est enlevé chirurgicalement (par exemple, lors d'une opération de la cataracte) et éventuellement remplacé par une lentille intraoculaire de chambre postérieure conventionnelle.

Depuis quelques années sont apparues sur le marché des lentilles intraoculaires de chambre postérieure dotées d'un filtre supplémentaire de la lumière bleue (entre 400 et 500 nm de longueur d'onde) par rapport à une lentille conventionnelle avec uniquement un filtre UV (exemple, Acrysof Natural par Alcon) bloquant les longueurs d'onde inférieures à 400 nm. Ces lentilles de couleur jaune ou orange miment le cristallin adulte en filtrant la lumière visible inférieure à 400 nm et en réduisant fortement la transmission de la lumière bleue comprise entre 400 et 500 nm de haute énergie (moins de 50 % de transmission pour les longueurs d'onde inférieures à 450 nm). Ces implants avec chromophore chimique restaurent donc la protection naturelle de l'oeil adulte contre les effets délétères pour la rétine de la lumière bleue. En outre, l'implantation d'une lentille synthétique qui mime le cristallin naturel adulte permet au patient de recouvrer une vision correspondant à son âge, sans être perturbé par une vision juvénile et trop blanche, comme c'est le cas en présence d'une lentille conventionnelle.

Les lentilles filtrant les "bleus" toxiques actuellement disponibles sont constituées de matériaux acryliques ou de silicones et sont de nature hydrophobe, c'est-à-dire que leur taux de prise d'eau à l'équilibre est inférieur à 5 % en poids. Le chromophore utilisé ou colorant possède en fait une entité polymérisable. De cette façon, l'agent colorant copolymérise avec les autres monomères impliqués dans la formulation du matériau de constitution de la lentille, ce qui assure sa stabilité chimique.

Dans la plupart des cas, le chromophore utilisé est de type phényl-azo-phényle (Ph-N=N-Ph) (US-5470932, US-5662707, JP-1299560). Le chromophore est fixé sur l'entité polymérisable (insaturation) de type (méth)acrylique ou (méth)acrylamide, soit directement (JP-1299560) via une liaison ester ou amide, soit via un "espaceur" constitué d'une courte chaîne hydrocarbonée (US-6242551, US-6326448). Cet "espaceur" est alors fixé directement sur la partie colorante (liaison carbone-carbone) ou, alternativement, via une liaison amine (-N-). Au moins un des deux groupes phényle du chromophore est généralement porteur d'au moins un groupe substituant, autre que l'entité polymérisable ou son « espaceur » de fixation audit chromophore.

Les lentilles colorées en jaune actuellement disponibles et filtrant les « bleus » toxiques souffrent d'un inconvénient majeur. Si le fait de filtrer une part de la composante bleue de la lumière visible qui pénètre dans l'oeil protège la rétine (on parle alors de photo-protection), cet effet protecteur s'accompagne d'une diminution de la qualité de la vision particulièrement dans des conditions de faible luminosité (inférieure à 2,5 candelas/mètre carré). La photo-protection est donc obtenue aux dépens de la photo-réception, c'est-à-dire la sensibilité visuelle.

L'implant coloré qui comprend un agent chromophore entraîne dans ces conditions particulières une réduction plus ou moins importante de la perception des couleurs et de la sensibilité aux contrastes selon le taux de chargement et l'efficacité du filtre chromophore utilisé. En effet, au contraire de ce qui se passe en vision de jour dite photopique (luminosité de l'ordre de 85 candelas/mètre carré), les courtes longueurs d'onde de la lumière visible (bleu-violet) jouent un rôle prépondérant dans la sensibilité visuelle, lorsque la luminosité est moindre (vision mésopique, 2,5 candelas/mètre carré), voire quasi absente (vision scotopique). La lumière bleue assure ainsi jusqu'à 35 % de la sensibilité visuelle en conditions scotopiques et plus de 50 % de l'illumination mélanopsinique.

D'autre part, une autre difficulté réside dans l'élaboration d'un additif hydrolytiquement stable qui soit adapté aux implants hydrophiles (avec un taux de prise d'eau à l'équilibre supérieur à 5 % massique). En effet, contrairement aux lentilles dites hydrophobes, les matériaux hydrophiles absorbent une quantité d'eau plus ou moins importante, selon leur nature (de l'ordre de 26 % massique). Cette eau d'hydratation pénètre dans le coeur de la matière et serait à même d'hydrolyser l'additif chromophore. Ceci est particulièrement vrai lors de la stérilisation à la vapeur de la lentille, processus dont les conditions de pression et de température (121 °C) sont particulièrement sévères. Dans ces conditions, et dans le cas d'une stabilité hydrolytique insuffisante, la lentille va voir sa couleur jaune diminuer rapidement, de la même façon que l'effet protecteur recherché contre la lumière bleue toxique, sans évoquer l'éventuelle toxicité des composés libérés de l'implant. Les filtres chromophores de l'état de l'art pour lesquels l'entité polymérisable est liée directement au groupe chromophore via un ester et dans une moindre mesure un amide sont particulièrement sensibles à l'hydrolyse, ce qui rend ce type de colorant polymérisable incompatible avec les LIOs hydrophiles et/ou stérilisées en phase vapeur.

La présente invention a pour but de mettre au point une composition polymère pour lentille intraoculaire permettant d'éviter les inconvénients précités. Avantageusement cette composition permettra la fabrication de lentilles, de préférence hydrophiles.

Pour résoudre ces problèmes, on a prévu suivant l'invention, une composition polymère pour lentille intraoculaire comprenant au moins un agent chromophore jaune copolymérisé, tel qu'indiqué au début, cette composition étant caractérisée en ce que le premier isomère de l'agent chromophore susdit présente un premier pouvoir de filtration de lumière bleue et le deuxième isomère de l'agent chromophore susdit présente un deuxième pouvoir de filtration de lumière bleue supérieur au premier,
en ce que, à une température de l'ordre de celle d'un oeil, le premier isomère est capable de transiter sous la forme du deuxième isomère en présence d'une irradiation de lumière ultraviolette et le deuxième isomère est capable de transiter sous la forme du premier isomère en l'absence d'une telle irradiation, et
en ce que, en présence de lumière, la composition présente un rapport variable entre le premier isomère et le deuxième isomère susdits.

On obtient de cette façon une composition dont le filtre « bleu » peut être ajusté automatiquement et de façon réversible aux conditions de luminosité. La part de lumière bleue transmise par une lentille fabriquée avec une telle composition varie de façon inversement proportionnelle à l'intensité de l'ultraviolet proche incident associé au spectre solaire et ce de façon réversible, grâce à une variation du rapport entre les deux isomères dans la composition, en présence d'une lumière variable. Sous une lumière intense, lorsque la part d'ultraviolet proche (400-500 nm) de la lumière est importante, le filtre est exacerbé et atténue fortement la fraction de rayons bleus délétères pour la rétine qui pourrait pénétrer dans l'oeil. Lorsque la luminosité baisse, le filtre perd progressivement et automatiquement une partie de son efficacité en laissant passer une part croissante des longueurs d'onde concernées (400-500 nm), et ce au profit de la perception des couleurs et de la sensibilité aux contrastes dans ces conditions particulières de luminosité réduite. Cette adaptabilité du filtre chromophore aux conditions d'éclairement est de préférence à 100 % réversible. Cette invention combine donc au sein d'une même lentille la qualité de vision associée à un implant conventionnel (photo-réception) et le pouvoir protecteur d'une LIO jaune (photo-protection), lorsque les conditions d'éclairement rendent nécessaires cet effet protecteur.

Selon une forme de réalisation suivant l'invention, la composition polymère comprend
- au moins un monomère acrylique et/ou méthacrylique, à l'état polymérisé,
- ledit au moins un agent chromophore jaune copolymérisé avec ledit au moins un monomère acrylique et/ou méthacrylique, cet agent chromophore comportant
   a) un groupe acrylate ou méthacrylate,
   b) une partie chromophore de type phényl-azo-phényle (Ph-N=N-Ph), qui se présente sous forme trans et/ou cis, et
   c) un espaceur constitué d'une chaîne hydrocarbonée linéaire ou ramifiée, contenant de 1 à 10 atomes de carbone et éventuellement interrompue par de l'oxygène, cet espaceur étant relié, d'une part, à une fonction ester du groupe acrylate ou méthacrylate susdit et, d'autre part, à la partie chromophore par une fonction éther (-O-),
- et éventuellement un agent anti-UV filtrant des longueurs d'onde inférieures à 400 nm.

La présente invention prévoit ainsi une composition photoisomérisable présentant un agent chromophore polymérisable qui, incorporé au moment de la polymérisation dans une composition de lentille, confère à cette dernière la capacité de filtrer la lumière bleue hautement énergétique, même si cette composition donne lieu à une lentille hydrophile et/ou stérilisée en phase vapeur, et dont le filtre est ajustable aux conditions d'éclairement combinant ainsi l'effet protecteur et la qualité de la vision recherchés.

Ledit espaceur est constitué d'une chaîne hydrocarbonée linéaire ou ramifiée, rattachée à ladite partie chromophore dont les groupes phényle sont disposés l'un par rapport à l'autre en position cis et/ou en position trans, de façon à rendre la composition photoisomérisable.

L'agent chromophore mis au point dans le cadre de cette invention, outre les caractéristiques de transmission optique désirées par l'application, jouit d'une très grande stabilité hydrolytique, et ce même en conditions de stérilisation à la vapeur (121 °C, 20 minutes).

La structure chimique générale de l'agent chromophore qui fait l'objet de la présente invention est la suivante :
Partie chromophore-O-espaceur-monomère.
La partie chromophore de la molécule de l'agent chromophore appartient à la catégorie des phényl-azo-phényles (Ph-N=N-Ph). Les groupes phényle peuvent éventuellement être substitués par des radicaux non électro-attracteurs et stériquement peu encombrants, tels que par exemple OH ou NH₂. De préférence seul un des groupes phényle porte un tel substituant et, tout à fait préférentiellement, aucun groupe phényle n'en porte.

Le monomère est de type acrylique ou méthacrylique. Il est lié au colorant via une chaîne hydrogéno-carbonée contenant de 1 à 10 atomes de carbone, appelée espaceur, éventuellement interrompue par de l'oxygène. La fonction ester du monomère est mise à profit pour fixer l'espaceur, alors que ce dernier est lié à la partie chromophore via une fonction éther (-O-).

Suivant une forme avantageuse de réalisation de l'invention, ledit au moins un agent chromophore répond à la formule I suivante 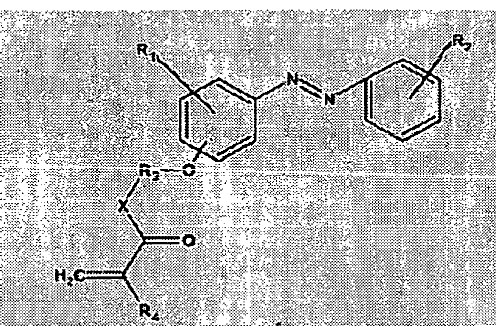 dans laquelle
R₁ et R₂ représentent chacun, indépendamment l'un de l'autre, H, OH ou NH₂,
R₃ représente ladite chaîne hydrocarbonée de l'espaceur,
R₄ représente H ou CH₃, et
X représente O, NH ou NR₅,
R₅ représentant une chaîne hydrocarbonée comprenant jusqu'à 10 atomes de C.

Un exemple de cet agent chromophore polymérisable est le méthacrylate de 2-(4-phénylazophénoxy)éthyle (acronyme : PAEM) dont la formule est reportée ci-dessous. Un synonyme est l'éthyl-2-méthyl-2-[4-(phénylazo)phénoxy]-propénoate (CAS N°: 140191-27-1, formule moléculaire: C18H18N2O3, masse moléculaire : 310,35). Il s'agit d'un composé solide de couleur orange-jaune.

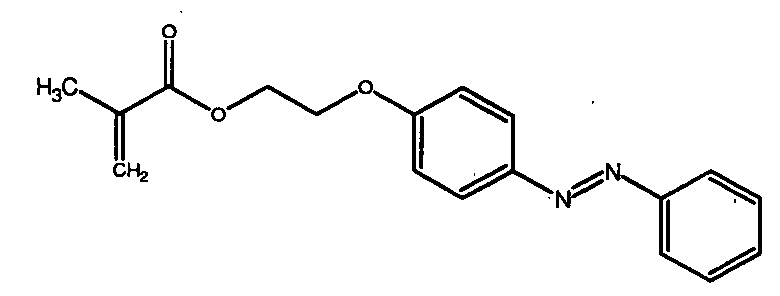

La synthèse de cet additif est très simple et est décrite notamment dans Jian Ping Chen et al., Journal of Polymer Science : Part A : Polymer Chemistry, Vol. 35, 9-16 (1997). L'identité chimique du composé obtenu a été vérifiée par différentes analyses dont les résultats sont reportés dans le tableau ci-dessous.

### Données de caractérisation de l'agent chromophore jaune synthétisé de type méthacrylate de 2-(4-phénylazophénoxv)éthyle (acronyme : PAEM)

| | |
|---|---|
| RMN du proton (CDCl₃, 200 MHz) | 1,96 ppm (s, 3H), 4,31 ppm (t, 2H), 4,53 ppm (t, 2H), 5,60 ppm (s, 1 H), 6,16 ppm (s, 1 H), 7,03 ppm (d, 2H), 7,47 ppm (m, 3H) et 7,89 ppm (m, 4H). |
| RMN du carbone 13 (CDCl₃, 50 MHz) : | ¹³C-NMR (CDCl₃, 50 MHz) : 18,5 (CH₃), 63,1 (CH₂), 66,4 (CH₂), 115,0 (2 CH aro), 122,8 (2 CH aro), 125,0 (2 CH aro), 126,4 (=CH₂), 129,2 (2 CH aro), 130,7 (CH aro), 136,1 (C=), 147,4 (C aro), 152,9 (C aro), 161,2 (C aro), 167,5 (CO₂). |
| Point de fusion | 68-70 (°C) |
| Infrarouge (FTIR) | 3039, 2963, 1717, 1706, 1633, 1601, 1582, 1503, 1463, 1443, 1413, 1400, 1379, 1325, 1300, 1251, 1185, 1141, 1066, 1042, 1000, 950, 925, 884, 831, 799, 720, 695, 686, 658, 632, 602, 550, 528, 516, 502 et 521 cm⁻¹ |
| Analyse élémentaire % | C (69.55), H (5.87), N (8.93) |
| HPLC | Temps de rétention de 17,05 min et pureté de 97, 5% |
| GC/MS | Temps de rétention de 21,11 minutes et pureté 100% |

Suivant une forme de réalisation particulière de l'invention, la teneur en ledit agent chromophore est inférieure à 0,25 % en poids de la composition globale. L'agent chromophore est de préférence incorporé à hauteur de 0,05-0,1 % en poids à la formulation d'une lentille hydrophile ou hydrophobe conventionnelle contenant en outre un agent anti-UV classique et un agent de réticulation. A titre d'exemples, des compositions chimiques de lentilles hydrophiles sont reportées dans le tableau ci-dessous.

| Monomères (*) | Teneur en eau |
|---|---|
| Lentilles intraoculaires souples hydrophiles (acryliques réticulés) (**) | |
| HEMA | 38 % |
| HEMA-co-MMA | 26 % |
| HEMA-co-EMA | 26 % |
| HOHEXMA-co-HEMA | 27 % |
| EOEMA-co-H EMA | 25 % |

| | |
|---|---|
| (*) HEMA : méthacrylate de 2-hydroxyéthyle; MMA : méthacrylate de méthyle; EMA : méthacrylate d'éthyle; HOHEXMA : méthacrylate d'hydroxyhexyle; EOEMA : méthacrylate d'éthoxyéthyle. (**) L'agent de réticulation est très souvent de type EGDMA ou diméthacrylate d'éthylène glycol. Un exemple d'agent anti-UV conventionnel est la 4-méthacryloxy-2-hydroxybenzophénone. | |

Afin d'étudier les caractéristiques de transmission d'une lentille hydrophile conventionnelle modifiée par l'agent chromophore qui fait l'objet de cette invention, de 0,05 à 0,1 % en poids de cet additif (PAEM) a été ajouté à la formulation d'un matériau hydrophile à 26 % contenant un agent anti-UV classique (voir tableau ci-dessus), l'implant conventionnel servant par ailleurs de lentille témoin. Le procédé de polymérisation du matériau est similaire à celui mis en jeu habituellement, de même que le mode de fabrication de la lentille qui en découle (fraisage-tournage, polissage, hydratation et stérilisation vapeur). Les protocoles expérimentaux sont reportés au sein des exemples 1 à 3.

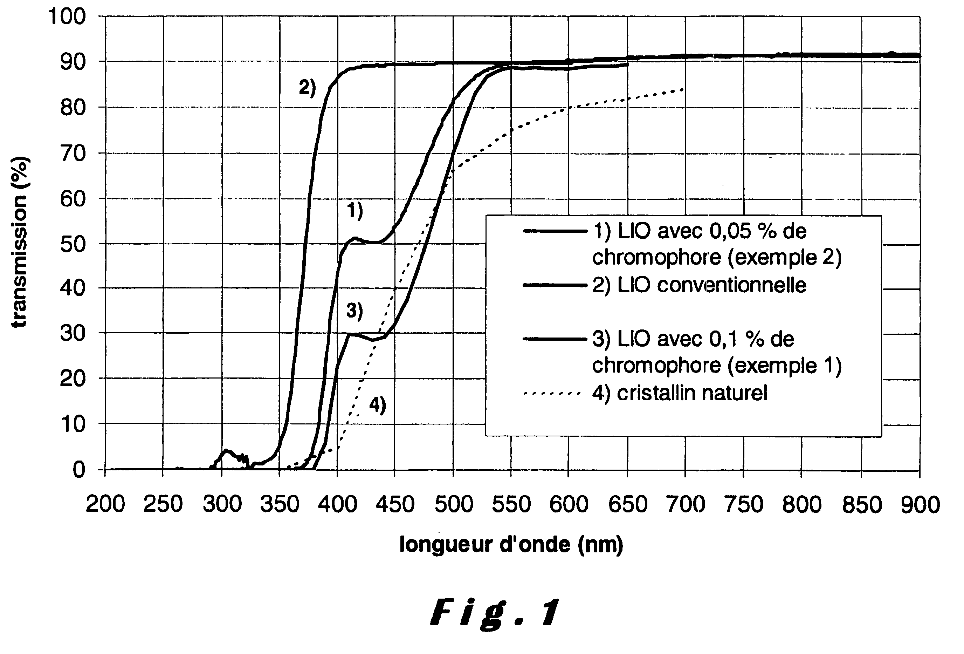

Afin d'éviter des artefacts de mesure lors de l'enregistrement des valeurs de transmission spectrales, des fac-similés de LIOs ont été produits prenant la forme de disques plats dont l'épaisseur centrale correspond à celle d'une LIO de 20 dioptres. Ces disques pré-hydratés ont été mesurés à l'aide d'un spectro-photomètre UV-visible (Perkin-Elmer Lambda 14 P) balayant des longueurs d'ondes entre 900 et 200 nm à la température ambiante. La figure 1 annexée montre que l'utilisation de l'agent chromophore étend le filtre de la lentille conventionnelle (bloquant uniquement les UV) vers les grandes longueurs d'onde en atténuant la transmission de la frange bleue de la lumière visible comprise entre 400 et 500 nm. Comme attendu, la part de lumière transmisse pour cette gamme de longueurs d'ondes diminue avec la quantité de chromophore ajouté à la formulation de la lentille hydrophile. En comparaison avec la lentille conventionnelle bloquant uniquement les UV (voir tableau, ci-dessous), la lentille jaune modifiée par 0,05 % en poids de chromophore polymérisable réduit graduellement la transmission de lumière relativement à la transmission de la lentille conventionnelle de 45 % (à 400 nm) et de 21 % (à 475 nm).

### Transmission optique en % des lentilles conventionnelles (20 D) et avec chromophore polymérisable de l'invention (exemple 2) à quatre longueurs d'onde.

| | 400 nm | 425 nm | 450 nm | 475 nm |
|---|---|---|---|---|
| Lentille conventionnelle à 26 % d'eau (1) | 87 | 89 | 89 | 90 |
| Lentille conventionnelle à 26 % d'eau modifiée par 0,05 % de chromophore (2) | 48 | 55 | 58 | 71 |
| Différence de transmission (T) T(1)-T(2) | 39 | 34 | 31 | 19 |
| Réduction de la transmission (T) pour la lentille avec chromophore (en % de la transmission de la lentille conventionnelle) [T(1)-T(2)]/T(1)(x100) | 45 | 38 | 35 | 21 |

Une partie de la lumière bleue de haute énergie est donc effectivement filtrée. A l'instar du cristallin naturel, la lentille agit donc comme un filtre protecteur et réduit le risque de lésions rétiniennes.

Afin de valider la stabilité hydrolytique de l'agent chromophore, des lentilles finales et stérilisées en phase vapeur (lentilles témoins) ont été soumises à un traitement drastique, avant de mesurer leurs caractéristiques spectrales. Le traitement utilisé est le suivant : une double stérilisation à la vapeur additionnelle dans une solution saline à 0,9 % de NaCl (121 °C, 20 minutes) (lentilles 1), suivie éventuellement d'une période d'incubation à 80°C pendant 13 jours dans de l'eau saline à 0,9 % de NaCl (lentilles 2). Les caractéristiques de transmission UV-visible des lentilles 1 et 2 sont similaires à celles des lentilles témoins, simplement stérilisées. L'additif est donc chimiquement stable en conditions d'hydrolyse. Les analyses de cytotoxicité et des produits provenant de la décomposition potentielle de l'agent chromophore réalisées directement sur le surnageant des implants après stérilisation et/ou après incubation à 80°C confirment cette situation.

La propriété intéressante de l'agent chromophore suivant l'invention est liée à sa capacité de photo-isomériser et ce de manière réversible. Les azobenzènes et leurs dérivés (dont l'objet de l'invention fait partie) existent dans une forme isomère géométrique trans ou cis.

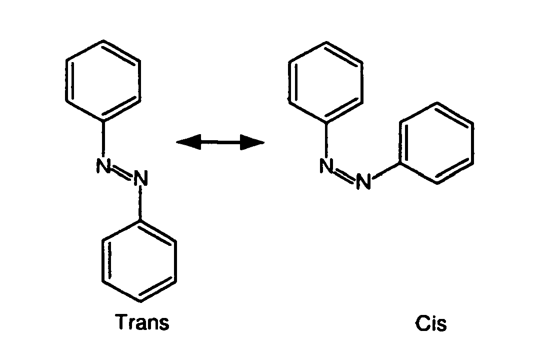

Ces isomères peuvent subir une transition isomérique réversible entre ces deux formes sous l'effet d'une irradiation ultraviolette (trans vers cis) ou d'un apport de chaleur (cis vers trans). La forme cis fait intervenir un angle dihédrique CNNC de 0° contre 180° pour la forme trans. Les groupes azoïques de la partie chromophore de l'agent chromophore suivant l'invention sont polymérisés dans leur forme thermodynamiquement favorable, c'est-à-dire la forme trans, caractérisée par deux pics d'absorbance, respectivement à 340 nm et 430 nm. Dans le cas de l'isomère cis, thermodynamiquement moins favorable, l'intensité du pic d'absorbance à 340 nm est inférieure, mais elle est supérieure à 430 nm.

Dès lors, après implantation dans l'oeil et en pleine intensité solaire (lumière qui contient une part d'UV : la portion des longueurs d'ondes comprises entre 300 et 400 nm, ultraviolet proche ou UVA, du spectre solaire représente environ 6,5 % de l'intensité totale dont 40 à 50 % sont absorbés par la cornée et l'humeur aqueuse en avant de la lentille implantée dans le sac capsulaire), les groupes azoïques de la partie chromophore de la lentille, initialement en configuration trans, vont subir progressivement une transition vers la forme cis. Dans cette forme cis, la filtration des bleus toxiques (400-500 nm) est plus efficace. L'atténuation du second pic d'absorption centré à 340 nm, lorsque l'on passe de la forme trans à cis, devrait s'accompagner d'une augmentation de la part des UV (longueur d'onde inférieure à 400 nm) qui pénètre dans l'oeil. C'est pourquoi il est préférable de prévoir en outre dans la composition suivant l'invention un agent anti-UV qui joue son rôle à ce niveau, en filtrant efficacement les rayons UV et ce indépendamment de la configuration géométrique du chromophore cis ou trans.

Lorsque l'intensité de la lumière solaire et la part des UVA qu'elle contient diminuent, les groupes azoïques de la partie chromophore, en configuration cis, vont retourner progressivement vers leur forme trans initiale et le filtre « bleu » sera atténué au profit d'une plus grande photo-réception et d'une sensibilité visuelle accrue.

Il s'agit d'un mécanisme de protection de la vision vis-à-vis des effets délétères des longueurs d'onde comprises entre 400 et 500 nm (lumière bleue) qui fait intervenir une isomérisation réversible trans-cis du chromophore introduit dans la lentille sous l'action des photons appartenant à l'ultraviolet proche entre 300 et 400 nm.

Ce comportement de photo-isomérisation a été simulé et validé « in vitro » en présence de lentilles constituées d'un matériau hydrophile à 26 % contenant un agent anti-UV classique et 0,1 % de chromophore polymérisable de type PAEM (exemple 1). Les caractéristiques de transmission spectrale des lentilles ont été mesurées entre 400 et 500 nm selon les conditions habituelles, mais après avoir soumis les implants aux traitements suivants :
1. (1) Irradiation des lentilles préhydratées immergées dans une solution aqueuse de NaCl à 0,9 % sous une lampe au mercure et pourvue d'un filtre dont le spectre d'émission UV est centré à 365 nm pendant au moins 5 minutes, avant de mesurer leurs transmissions optiques.
2. (2) Les lentilles traitées selon les conditions décrites au point (1), sont soumises à un post-traitement thermique à une température de l'ordre de celle d'un oeil, par exemple à 38°C durant au moins 100 minutes en absence d'irradiation UV, avant de mesurer leurs caractéristiques spectrales.

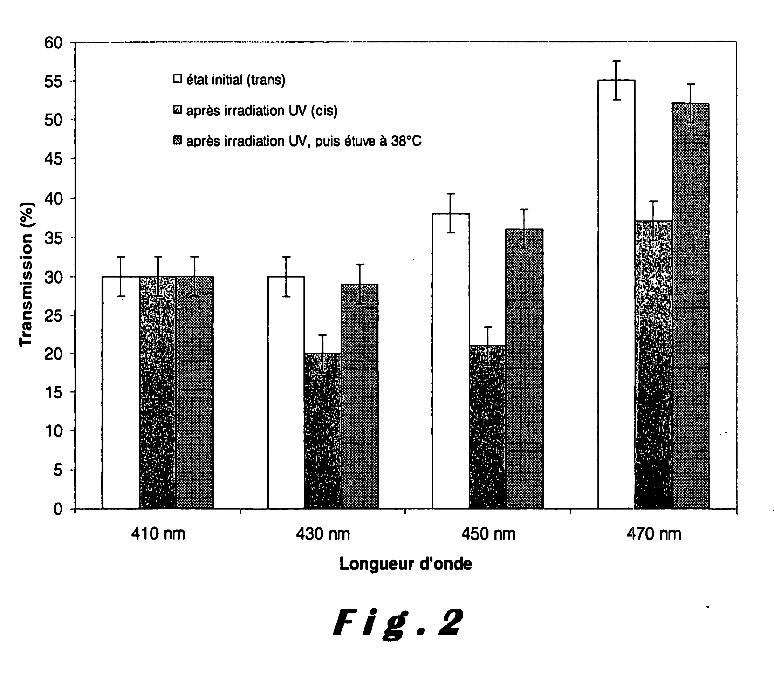

Les résultats sont reportés à la figure 2 annexée et montrent clairement que l'isomérisation « trans-cis-trans » des groupes chromophores a lieu comme décrit plus haut dans le texte. L'effet protecteur est renforcé suite à l'irradiation UV des implants durant au moins 5 minutes dans les conditions du test. Ce temps d'irradiation correspond à la mise en place d'un état stationnaire. Si l'on assimile l'effet protecteur à la réduction de la fraction de lumière transmise entre 400 et 500 nm par la lentille irradiée par rapport à la lentille avant irradiation, la protection de la rétine contre par exemple une longueur d'onde émise de 450 nm augmente de 45 % suite à l'irradiation de l'implant. Cela signifie que la transmission de la lumière bleue délétère peut être réduite de moitié lorsque le chromophore transite d'un isomère géométrique trans à un isomère cis sous l'effet d'un rayonnement intense et ce avec une cinétique élevée. Cette transition entre deux formes isomères aux caractéristiques de transmission spectrale différentes est par ailleurs réversible. Les lentilles recouvrent en effet leurs caractéristiques de transmission initiales, dès que l'irradiation cesse et après une période d'incubation à 38 °C d'au moins 100 minutes.

Le même test, réalisé avec des lentilles hydrophobes de l'état de l'art (Acrysof ® Natural IOL Model SN60AT, Alcon ®) constituées de deux monomères principaux (acrylate de phényléthyle et méthacrylate de phényléthyle) et modifiées par un chromophore de type (N-2[3-(2-méthylphénylazo)-4-hydroxyphényl]éthyl méthacrylamide) (US-A-5470932) greffé chimiquement, ne manifeste aucun changement des caractéristiques de transmission suite à une exposition prolongée aux UV, jusqu'à une heure.

La présente invention a également pour objet l'utilisation d'une composition suivant l'invention, pour la fabrication de lentilles intraoculaires, de préférence mais pas exclusivement de lentilles intraoculaires hydrophiles.

La présente invention est également relative à une lentille intraoculaire à base d'une composition suivant l'invention ou obtenue selon l'utilisation suivant l'invention. De préférence de telles lentilles présentent une absorbance de la lumière bleue qui diminue de moins de 10 % après trois stérilisations à la vapeur successives.

L'invention va à présent être décrite de manière plus détaillée à l'aide d'exemples donnés ci-dessous à titre non limitatif.

### Exemple 1 : Fabrication d'un lentille hydrophile

On combine du méthacrylate de 2-hydroxyéthyle (HEMA) avec du méthacrylate de méthyle (MMA) dans un rapport pondéral de 9/1 en présence de 0,2 % en poids d'amorceur de polymérisation de type azobis(isobutyronitrile) (AIBN), 2,9 % en poids d'un agent de réticulation conventionnel à base de diméthacrylate d'éthylène glycol (EGDMA), 1 % d'un agent anti-UV conventionnel de type 4-méthacryloxy-2-hydroxybenzophénone et 0,1 % en poids du chromophore polymérisable de type méthacrylate de 2-(4-phénylazophénoxy)éthyle (acronyme : PAEM) mentionné précédemment. Le volume total de la composition est d'environ 14 ml. Après homogénéisation dans un bain ultrasonique pendant 10 minutes à la température ambiante, la solution est filtrée sous 0,2 micromètre et transférée dans un ballon en verre pourvu d'un barreau magnétique et fermé par un bouchon. Ensuite, sous agitation, on fait barboter dans la solution du gaz argon ultrapur pendant 2 minutes et on soumet la solution à un vide dynamique pendant 2 minutes. Le cycle barbotage à l'argon/vide est répété au moins 3 fois. 2 ml de la solution sont ensuite transférés par l'utilisation d'une seringue dans des moules (en PTFE) saturés au préalable en argon. Le moule est alors placé dans un bain d'eau à température régulée et il est chauffé à 58°C pendant au moins 18 heures. Après cette étape de polymérisation, le moule est lentement refroidi à 50°C et maintenu à cette température pendant au moins 20 minutes. Le moule est ouvert et les pièces polymérisées sont recuites dans une étuve sous vide dynamique (0,001 Hg) à successivement 65 °C pendant 4 heures, 75 °C pendant 4 heures, 90°C pendant 24 heures et 120°C pendant 12 heures. On procède alors à un lent refroidissement à la température ambiante tout en maintenant le vide statique. Les parties centrales des pièces polymérisées sont ensuite découpées en disques d'épaisseur correspondant à l'épaisseur centrale d'une lentille d'une vergence de 20 dioptres et qui serait constituée du même matériau par une technique conventionnelle de coupe au tour. Les composés potentiellement toxiques, dont les monomères résiduels, sont extraits pendant 48 heures dans de l'eau ultrapure. Les disques sont ensuite placés dans des flacons individuels remplis d'une solution saline à 0,9 % en poids de NaCl.

L'indice de réfraction des disques hydratés (1,46) est déterminé sur un réfractomètre.

Pour déterminer la teneur en eau à l'équilibre, les disques préalablement hydratés dans la solution saline sont séchés superficiellement à l'aide d'un tissu absorbant et pesés sur une balance analytique (précision : 0,1 mg). Ils sont ensuite séchés dans une étuve à 120°C sous vide dynamique, jusqu'à poids constant. La teneur en eau à l'équilibre pour chaque disque est alors déterminée par gravimétrie en utilisant l'équation suivante :
Teneur en eau à l'équilibre = 100 x [(masse hydratée - masse déshydratée) / masse déshydratée ]
La teneur en eau à l'équilibre mesurée dans ces conditions est d'environ 26 % en poids.

### Exemple 2 : Fabrication d'une lentille hydrophile

Des lentilles sont fabriquées et caractérisées selon le protocole décrit dans l'exemple 1, mais avec 0,05 % en poids d'agent chromophore polymérisable de type méthacrylate de 2-(4-phénylazophénoxy)éthyle au lieu de 0,1 %. Les indices de réfraction (1,46 dans l'état hydraté) et la prise d'eau à l'équilibre de l'ordre de 26 % en masse restent inchangés par rapport au matériau décrit dans l'exemple 1. Comme attendu, la courbe expérimentale reportée à la figure 1 en annexe est déplacée vers des valeurs de transmission supérieure par rapport au matériau contenant deux fois plus d'agent chromophore.

### Exemple 3 : Fabrication d'une lentille hydrophile

Des lentilles sont fabriquées et caractérisées selon le protocole décrit dans l'exemple 1, mais en modifiant la formulation du matériau. On combine ici de l'acrylate de 2-phénoxyéthyle (EGPEA) et du méthacrylate de 2-hydroxyéthyle (HEMA) en un rapport pondéral de 1,7 en présence de 0,2 % en poids d'amorceur azobis(isobutyronitrile) (AIBN), de 0,05 % en poids d'agent chromophore polymérisable de type méthacrylate de 2-(4-phénylazophénoxy)éthyle,de 0,5 % d'un agent anti-UV de type acrylate de 2-(4-benzoyl-3-hydroxyphénoxy)éthyle (BHPEA) et de 8 % en poids d'un agent de réticulation polymère à poids moléculaire élevé, c'est-à-dire du diméthacrylate de polypropylène glycol (PPGDMA, poids moléculaire environ 560). Les copolymères synthétisés ont été découpés en disques comme dans l'exemple 1, mais en utilisant une technique plus appropriée en raison de la flexibilité du matériau à la température ambiante. Une technique de découpe au tour est mise à profit, mais la pièce à usiner est fixée sur une plaque de fixation rotative (broche) par un très mince film d'eau congelée. La plaque de fixation a été préalablement pulvérisée par de l'eau qui a été congelée par un fluide refroidi par un tube vortex utilisant de l'air comprimé. En utilisant cette technique, la cire, qui est habituellement utilisée pour fixer le disque sur la broche, n'est pas nécessaire et la plaque de fixation est maintenue à une gamme de températures basse pendant tout le processus de découpe.

La teneur en eau à l'équilibre des disques polymérisés et usinés est d'environ 6,5 % en poids, l'indice de réfraction d'environ 1,52. La température de transition vitreuse (Tv) des disques est mesurée par calorimétrie à balayage différentiel (DSC) avec une rampe de 10°C par minute et elle est mesurée au point central de la transition de la courbe de flux thermique. Les valeurs expérimentales de Tv sont de 23 et de 9°C dans l'état anhydre et hydraté, respectivement. La dureté Shore A des disques hydratés déterminée à la température ambiante avec un duromètre à 15 secondes est de 49.

Les disques pré-hydratés et séchés superficiellement sous un léger flux d'air sec ont été mesurés à l'aide d'un spectro-photomètre UV-visible (Perkin-Elmer Lambda 14 P) balayant des longueurs d'ondes entre 900 et 200 nm à la température ambiante.

Il doit être entendu que la présente invention n'est en aucune façon limitée aux formes de réalisation décrites ci-dessus et que bien des modifications peuvent y être apportées sans sortir du cadre des revendications annexées.

## Revendications

1. Composition polymère pour lentille intraoculaire comprenant au moins un agent chromophore jaune copolymérisé, constitué d'un isomère et d'un deuxième isomère et filtrant au moins une part de lumière bleue,
**caractérisé en ce que** le premier isomère de l'agent chromophore susdit présente un premier pouvoir de filtration de lumière bleue et le deuxième isomère de l'agent chromophore susdit présente un deuxième pouvoir de filtration de lumière bleue supérieur au premier,
**en ce que**, à une température de l'ordre de celle d'un oeil, le premier isomère est capable de transiter sous la forme du deuxième isomère en présence d'une irradiation de lumière ultraviolette et le deuxième isomère est capable de transiter sous la forme du premier isomère en l'absence d'une telle irradiation, et
**en ce que**, en présence de lumière, la composition présente un rapport variable entre le premier isomère et le deuxième isomère susdits.

2. Composition polymère suivant la revendication 1, **caractérisée en ce que** l'agent chromophore constitué des deux isomères précités filtre au moins une part de lumière bleue entre 400 et 500 nm et **en ce qu'**elle comporte en outre un agent anti-UV filtrant des longueurs d'onde inférieures à 400 nm.

3. Composition polymère suivant l'une des revendications 1 et 2, **caractérisé en ce qu'**elle comprend
- au moins un monomère acrylique et/ou méthacrylique, à l'état polymérisé,
- ledit au moins un agent chromophore jaune copolymérisé avec ledit au moins un monomère acrylique et/ou méthacrylique, cet agent chromophore comportant
a) un groupe acrylate ou méthacrylate,
b) une partie chromophore de type phényl-azo-phényle (Ph-N=N-Ph), qui se présente sous forme trans et/ou cis, et
c) un espaceur constitué d'une chaîne hydrocarbonée linéaire ou ramifiée, contenant de 1 à 10 atomes de carbone et éventuellement interrompue par de l'oxygène, cet espaceur étant relié, d'une part, à une fonction ester du groupe acrylate ou méthacrylate susdit et, d'autre part, à la partie chromophore par une fonction éther (-O-), et
- éventuellement un agent anti-UV filtrant des longueurs d'onde inférieures à 400 nm.

4. Composition suivant l'une des revendications 1 à 3, **caractérisée en ce que** ledit agent chromophore répond à la formule suivante 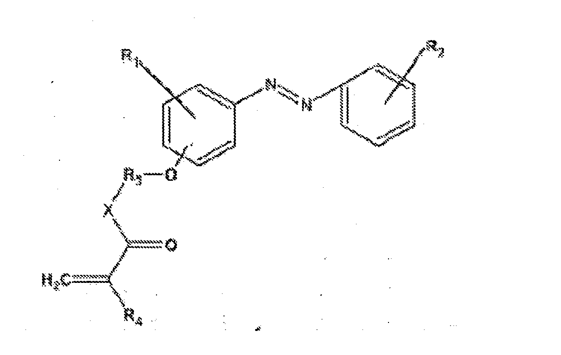 dans laquelle
R₁ et R₂ représentent, indépendamment l'un de l'autre, H, OH ou NH₂,
R₃ représente ladite chaîne hydrocarbonée de l'espaceur,
R₄ représente H ou CH₃, et
X représente O, NH ou NR₅,
R₅ représentant une chaîne hydrocarbonée comprenant jusqu'à 10 atomes de C.

5. Composition suivant l'une des revendications 1 à 4, **caractérisée en ce que** l'agent chromophore est du méthacrylate de 2-(4-phénylazophénoxy)-éthyle (PAEM).

6. Composition suivant l'une quelconque des revendications 1 à 5, **caractérisée en ce qu'**elle comporte une teneur en ledit agent chromophore inférieure à 0,25 % en poids.

7. Composition suivant l'une quelconque des revendications 3 à 6, **caractérisée en ce que** ledit au moins un monomère acrylique et/ou méthacrylique est hydrophile.

8. Composition suivant l'une quelconque des revendications 3 à 6, **caractérisée en ce que** ledit au moins un monomère acrylique et/ou méthacrylique est hydrophobe.

9. Utilisation d'une composition suivant l'une quelconque des revendications 1 à 8, pour la fabrication de lentilles intraoculaires.

10. Lentille intraoculaire à base d'une composition suivant l'une quelconque des revendications 1 à 8, et/ou telle qu'obtenue selon l'utilisation suivant la revendication 9.

## Claims

1. Polymer composition for an intraocular lens comprising at least one copolymerised yellow chromophore agent, consisting of a first isomer and a second isomer and filtering at least part of the blue light,
**characterised in that** the first isomer of the aforementioned chromophore agent has a first blue light filtration capacity and the second isomer of the aforementioned chromophore agent has a second blue light filtration capacity greater than the first,
**in that**, at a temperature of around that of an eye, the first isomer is capable of transiting in the form of the second isomer in the presence of an ultraviolet light irradiation and the second isomer is capable of transiting in the form of the first isomer in the absence of such irradiation, and
**in that**, in the presence of light, the composition has a variable ratio between the aforementioned first isomer and second isomer.

2. Polymer composition according to claim 1, **characterised**
**in that** the chromophore agent consisting of the aforementioned two isomers filters at least part of the blue light between 400 and 500 nm and in that it also comprises an anti-UV agent filtering wavelengths of less than 400 nm.

3. Polymer composition according to one of claims 1 and 2, **characterised in that** it comprises
- at least one acrylic and/or methacrylic monomer, in the polymerised state,
- the said at least one yellow chromophore agent copolymerised with the said at least one acrylic and/or methacrylic monomer, this chromophore agent comprising
a) an acrylate or methacrylate group,
b) a chromophore part of the phenyl-azo-phenyl type (Ph-N=N-Ph), which is in trans and/or cis form, and
c) a spacer consisting of a linear or branched hydrocarbon chain, containing 1 to 10 carbon atoms and possibly interrupted by oxygen, this spacer being connected firstly to an ester function of the aforementioned acrylate or methacrylate group and secondly to the chromophore part by an ether function (-O-), and
- possibly an anti-UV agent filtering wavelengths below 400 nm.

4. Composition according to one of claims 1 to 3,
**characterised in that** the said chromophore agent complies with the following formula 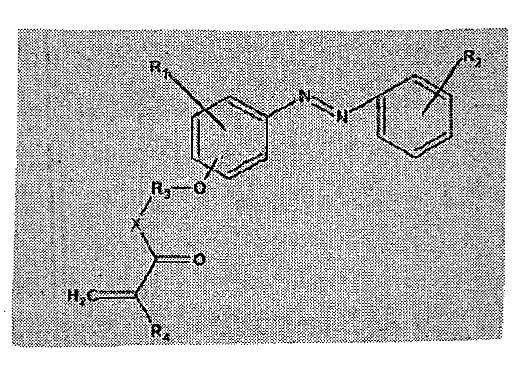 in which
R₁ and R₂ represent, independently of each other, H, OH or NH₂,
R₃ represents the said hydrocarbon chain of the spacer,
R₄ represents H or CH₃, and
X represents O, NH or NR₅,
R₅ representing a hydrocarbon chain comprising up to 10 carbon atoms.

5. Composition according to one of claims 1 to 4, **characterised in that** the chromophore agent is 2-(4-phenylazophenoxy)-ethyl methacrylate (PAEM).

6. Composition according to any one of claims 1 to 5, **characterised in that** it contains a proportion of the said chromophore agent of less than 0.25% by weight.

7. Composition according to any one of claims 3 to 6, **characterised in that** the said at least one acrylic and/or methacrylic monomer is hydrophilic.

8. Composition according to any one of claims 3 to 6, **characterised in that** the said at least one acrylic and/or methacrylic monomer is hydrophobic.

9. Use of a composition according to any one of claims 1 to 8, for manufacturing intraocular lenses.

10. Intraocular lens based on a composition according to any one of claims 1 to 8, and/or as obtained according to the use in accordance with claim 9.

## Patentansprüche

1. Polymerzusammensetzung für intraokulare Linsen, umfassend mindestens ein kopolymerisiertes gelbes farbgebendes Mittel, das aus einem ersten Isomer und einem zweiten isomer besteht und mindestens einen blauen Lichtanteil filtert,
**dadurch gekennzeichnet, dass** das erste Isomer des oben genannten farbgebenden Mittels ein erstes Blaulicht-Filtervermögen aufweist und das zweite Isomer des oben genannten farbgebenden Mittels ein zweites Blaulicht-Filtervermögen aufweist, das größer ist als das erste,
dass bei einer Temperatur wie die eines Auges das erste Isomer in der Lage ist, in die Form des zweiten Isomers überzugehen, wenn eine Bestrahlung mit ultraviolettem Licht vorliegt, und das zweite Isomer in der Lage ist, in die Form des ersten Isomers überzugehen, wenn eine derartige Bestrahlung nicht vorliegt, und
dass die Zusammensetzung bei Vorhandensein von Licht ein variables Verhältnis zwischen den ersten und zweiten oben genannten Isomeren aufweist.

2. Polymerzusammensetzung nach Anspruch 1, **dadurch gekennzeichnet, dass** das aus den beiden zuvor genannten Isomeren bestehende farbgebende Mittel mindestens einen blauen Lichtanteil zwischen 400 und 500 nm filtert, und dass sie ferner ein UV-Schutzmittel umfasst, das Wellenlängen von weniger als 400 nm filtert.

3. Polymerzusammensetzung nach einem der Ansprüche 1 und 2, **dadurch gekennzeichnet, dass** sie folgendes umfasst:
- mindestens ein Acrylmonomer und/oder Methacrylmonomer im polymerisierten Zustand,
- das mindestens eine gelbe farbgebendes Mittel, das mit dem mindestens einen Acryl- und/oder Methacrylmonomer kopolymerisiert wird, wobei dieses farbgebende Mittel folgendes umfasst:
a) eine Acrylat- oder Methacrylatgruppe,
b) einen Chromophorteil der Art Phenylazophenyl (Ph-N=N-Ph), der als Trans- und/oder Cis-Form vorliegt, und
c) einen Abstandhalter, der aus einer geraden oder verzweigten Kohlenwasserstoffkette besteht, die 1 bis 10 Kohlenstoffatome enthält und gegebenenfalls durch Sauerstoff unterbrochen wird, wobei dieser Abstandhalter einerseits mit einer Esterfunktion der oben genannten Acrylat- oder Methacrylatgruppe und andererseits mit dem Chromophorteil über eine Etherfunktion (-O-) verbunden ist, und
- gegebenenfalls ein UV-Schutzmittel, das Wellenlängen von weniger als 400 nm filtert.

4. Zusammensetzung nach einem der Ansprüche 1 bis 3, **dadurch gekennzeichnet, dass** das farbgebende Mittel folgender Formel entspricht: 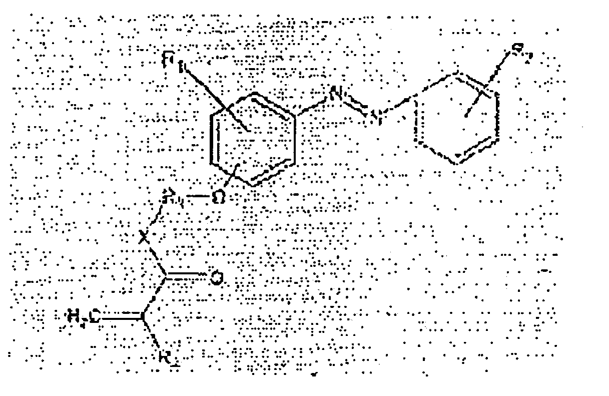 wobei
R₁ und R₂ unabhängig voneinander für H, OH oder NH₂ stehen,
R₃ für die Kohlenwasserstoffkette des Abstandshalters steht,
R₄ für H oder CH₃ steht und
X für O, NH oder NR₅ steht,
wobei R₅ für eine Kohlenwasserstoffkette steht, die bis zu 10 C-Atome umfasst.

5. Zusammensetzung nach einem der Ansprüche 1 bis 4, **dadurch gekennzeichnet, dass** das farbgebende Mittel 2-(4-Phenylazophenoxy)-ethyl-Methacrylat (PAEM) ist.

6. Zusammensetzung nach einem der Ansprüche 1 bis 5, **dadurch gekennzeichnet, dass** sie einen Gehalt an farbgebenden Mittel von weniger als 0,25 Gewichtsprozent umfasst.

7. Zusammensetzung nach einem der Ansprüche 3 bis 6, **dadurch gekennzeichnet, dass** das mindestens eine Acryl- und/oder Methacrylmonomer hydrophil ist.

8. Zusammensetzung nach einem der Ansprüche 3 bis 6, **dadurch gekennzeichnet, dass** das mindestens eine Acryl- und/oder Methacrylmonomer hydrophob ist.

9. Verwendung einer Zusammensetzung nach einem der Ansprüche 1 bis 8 zur Herstellung von intraokularen Linsen.

10. Intraokulare Linse auf der Grundlage einer Zusammensetzung nach einem der Ansprüche 1 bis 8 und/oder so wie sie gemäß der Verwendung nach Anspruch 9 gewonnen wird.
